(19) **Europäisches Patentamt**

**European Patent Office**

**Office européen des brevets**

(11) **EP 1 426 064 A1**

(12) **EUROPEAN PATENT APPLICATION**
published in accordance with Art. 158(3) EPC

(43) Date of publication:
09.06.2004 Bulletin 2004/24

(51) Int Cl.7: **A61L 2/18**, A61L 2/10

(21) Application number: 02762803.1

(22) Date of filing: 19.08.2002

(86) International application number:
PCT/JP2002/008341

(87) International publication number:
WO 2003/015834 (27.02.2003 Gazette 2003/09)

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: 20.08.2001 JP 2001248659
14.08.2002 JP 2002236242

(71) Applicant: **MENICON CO., LTD.**
**Nagoya-shi, Aichi 460-0006 (JP)**

(72) Inventors:
• NAKADA, Kazuhiro c/o Menicon Co Ltd
Kasuagai-shi Aichi-ken (JP)
• SAKANISHI, Kotaro c/o Menicon Co,Ltd
Kasugai-shi Aichi-ken (JP)
• Sugasawa, Noriko c/o Menicon Co Ltd,
Kasugai-shi Aichi-ken (JP)
• SUZUKI, Hiroaki c/o Menicon Co Ltd
Kasugai-shi Aichi-ken (JP)

(74) Representative: **Paget, Hugh Charles Edward**
**MEWBURN ELLIS**
**York House**
**23 Kingsway**
**London WC2B 6HP (GB)**

(54) **DISINFECTION METHOD**

(57) It is an object of the invention to provide a simple disinfecting method for assuring an excellent disinfecting effect. The present method uses a disinfecting liquid in which a hydrogen peroxide is included in an aqueous medium. The disinfecting liquid is accommodated in a suitable container such that the disinfecting liquid accommodated in the container has a depth of 3-10 mm, and the disinfecting liquid in which an object to be disinfected is immersed is irradiated with a light having a wavelength of 280-385 nm, such that the light is incident on the disinfecting liquid at least in a direction of the depth of the disinfecting liquid, so that the object immersed in the disinfecting liquid is disinfected.

EP 1 426 064 A1

**Description**

TECHNICAL FIELD

The present invention relates in general to a disinfecting method, and more particularly to such a method for disinfecting, in particular, medical instruments such as contact lenses and contact mirrors for an ophthalmic use, by irradiating a disinfecting liquid including hydrogen peroxide, with a light having a predetermined wavelength, for thereby providing an excellent disinfecting effect while eliminating a neutralizing treatment for neutralizing the hydrogen peroxide after disinfection.

BACKGROUND ART

Conventionally, contact lenses are classified into non-water-contained contact lenses and water-contained contact lenses, or hard contact lenses and soft contact lenses. During a long period of use of the contact lenses, microorganisms such as bacteria and fungi tend to adhere to and proliferate on the surface of the contact lenses while the contact lenses are stored after they have been removed from the eyes. Such microorganisms may cause infectious diseases, giving adverse influences on the eyes of the user. In view of the above, the contact lenses generally need to be disinfected before they are worn on the eyes. In particular, it is indispensable to disinfect the soft contact lenses since the microorganisms are likely to proliferate on the surface of the soft contact lenses more often than on the hard contact lenses, increasing a risk of causing the infectious diseases.

For disinfecting the contact lenses, there have been principally practiced a thermal disinfecting method using a suitable boiling and disinfecting device, and a chemical disinfecting method using a suitable germicide or a preservative. The thermal disinfecting method undesirably requires a time-consuming boiling operation to disinfect the contact lenses. Accordingly, in recent years, the chemical disinfecting method has been widely employed to disinfect the contact lenses.

In the chemical disinfecting method, a contact lens liquid agent containing a suitable germicide/preservative is used. The contact lenses are immersed in the liquid agent, so that the contact lenses are disinfected. As the germicide/preservative contained in the liquid agent, various chemical agents have been proposed. For instance, chlorhexidine, benzalkonium chloride and thimerosal are known. (Such chemical agents are disclosed in JP-A-52-109953, JP-A-62-153217 and JP-A-63-59960).

In order to obtain a sufficiently high disinfecting effect when the contact lenses are disinfected by the chemical disinfecting method, the germicide/preservative as the chemical agent such as chlorhexidine is contained in the liquid agent generally in a relatively high concentration. In this case, the germicide/preservative such as chlorhexidine is likely to be adsorbed on the contact lenses on a molecular level, deteriorating the wettability at the surface of the contact lenses, and causing a change in the properties and the configuration of the contact lenses. In some cases, the adsorption of the germicide/preservative on the contact lenses may undesirably cause various troubles with the eyes such as an allergy while the contact lenses are worn on the eyes. To keep the properties and the configuration of the contact lenses unchanged, and assure a high degree of safety with the eyes of the lens users, the contact lenses may be disinfected by using a liquid agent in which the germicide/preservative is contained in a relatively low concentration. In this case, however, the disinfecting effect to be exhibited by the liquid agent is inevitably lowered, causing contamination of the contact lenses by the microorganisms. Accordingly, when the contact lenses are disinfected by using such a conventional liquid agent, it is necessary to exercise an utmost care in adjusting the concentration of the germicide/preservative included in the liquid agent, making the disinfecting treatment of the contact lenses very cumbersome.

As the germicide/preservative used in the chemical disinfecting method, hydrogen peroxide is well known. To obtain the desired disinfecting effect, however, the hydrogen peroxide needs to be used generally in a concentration of about 3% (30,000 ppm). In addition, it is necessary to conduct a neutralizing treatment, after the disinfection of the contact lenses, for neutralizing the hydrogen peroxide remaining on the surface of the contact lenses or in the contact lenses, so that the hydrogen peroxide is decomposed and detoxified. The residual hydrogen peroxide remaining even in a small amount may cause a risk of giving serious troubles to the eyes of the user. Therefore, the neutralizing treatment has been conventionally conducted for completely decomposing and removing the residual hydrogen peroxide, by rinsing the disinfected contact lenses with a saline or physiological salt solution, or by using a metal catalyst such as platinum black, a reducing agent such as sodium sulfite, sodium thiosulfate, or pyruvic acid, or an enzyme catalyst such as catalase or peroxidase. Thus, the chemical disinfecting method using the hydrogen peroxide requires the neutralization treatment for neutralizing the residual hydrogen peroxide remaining after the disinfecting treatment, undesirably making the disinfecting treatment very cumbersome.

## DISCLOSURE OF THE INVENTION

The present invention was made in the background art situations described above. It is therefore an object of the invention to provide a disinfecting method which can be conducted in a simplified manner and which assures an excellent disinfecting effect.

The applicant of the present application proposed in JP-A-2001-190644 a disinfecting method which is different from the above-described conventional method using the germicide/preservative. Namely, the proposed method comprises: immersing a contact lens in a suspension obtained by dispersing fine particles of titanium oxide in an aqueous medium; and irradiating the suspension with a light. The proposed method utilizes the germicidal activity of the titanium oxide based on its photocatalytic action, for thereby attaining an excellent disinfecting effect. The inventors of the present invention have made a further study on the proposed disinfecting method, and have found that the disinfecting effect can be effectively improved while eliminating the neutralization treatment, by irradiating, under predetermined conditions, a disinfecting liquid merely including the hydrogen peroxide without the fine particles of titanium oxide being dispersed therein, with a light having a predetermined wavelength.

The present invention was made based on the findings described above. The present invention provides a method of disinfecting an object by using a disinfecting liquid in which hydrogen peroxide is included in an aqueous medium, characterized by comprising: accommodating the disinfecting liquid in a predetermined container such that the disinfecting liquid accommodated in the container has a depth of 3-10 mm; and irradiating the disinfecting liquid in which the object to be disinfected is immersed, with a light having a wavelength of 280-385 nm, such that the light is incident on the disinfecting liquid at least in a direction of the depth of the disinfecting liquid.

In the disinfecting method according to the present invention, the disinfecting liquid including the hydrogen peroxide as the germicide is accommodated in a container such that the disinfecting liquid has a relatively small depth of 3-10 mm, and the disinfecting liquid is irradiated with the light having the predetermined wavelength, such that the light is incident in the direction of the depth of the disinfecting liquid accommodated in the container. According to the present method, the hydrogen peroxide included and dissolved in the disinfecting liquid is uniformly and effectively irradiated with the light, so that the hydrogen peroxide which has been exposed to the light is effectively decomposed. Upon decomposition of the hydrogen peroxide, there are formed hydroxyradical ($\cdot$OH) having a strong oxidative action or power. Owing to the strong oxidative power of the hydroxyradical, the harmful microorganisms are effectively killed.

The present method assures an excellent disinfecting effect simply by irradiating the disinfecting liquid containing the hydrogen peroxide, with the light having the predetermined wavelength. By irradiating the disinfecting liquid with the light, the hydrogen peroxide which is harmful to a living subject or body is effectively and positively decomposed in a relatively short period of time. Even if the concentration of the hydrogen peroxide included in the disinfecting liquid is made relatively low, the present method assures a significantly high degree of germicidal efficacy while eliminating the neutralizing treatment for neutralizing the hydrogen peroxide after the disinfecting treatment.

In the present method, the disinfecting liquid is irradiated with the light having a wavelength of 280-385 nm, for thereby assuring a high degree of disinfecting effect without giving an adverse influence on the object to be disinfected.

In one preferred form of the disinfecting method according to the present invention, the light to be incident on the disinfecting liquid has an intensity of 0.3-1.0 mW/cm$^2$ at a dominant wavelength thereof. According to this arrangement, the hydrogen peroxide included in the disinfecting liquid is advantageously decomposed, for effectively disinfecting the object immersed in the disinfecting liquid.

In another preferred form of the disinfecting method according to the present invention, the hydrogen peroxide has a concentration of 1-500 ppm. The present invention advantageously assures an excellent germicidal efficacy even if the concentration of the hydrogen peroxide is relatively low.

In the disinfecting method according to the present invention, the disinfecting liquid may further include, as needed, a water-dispersible titanium oxide, a metal ion, a metal-EDTA complex, a surfactant, etc., so that the disinfecting liquid has intended properties given by those additives. More specifically described, the disinfecting liquid enjoys an improved disinfecting activity owing to the water-dispersible titanium oxide, an improved effect of promoting the decomposition of the hydrogen peroxide owing to the metal ion or the metal-EDTA complex, or a cleaning effect owing to the surfactant.

In the disinfecting liquid used in the present disinfecting method, the osmotic pressure is held preferably in a range of 250--350 mOsm. This arrangement assures a significantly high degree of safety with the living subject or body such as eyes while preventing the object to be disinfected such as contact lenses, from being adversely influenced.

In still another preferred form of the disinfecting method according to the present invention, the object to be disinfected includes, for example, the medical instruments such as contact lenses and contact mirrors for an ophthalmic use. In particular, the contact lenses are advantageously disinfected according to the present method.

BEST MODE FOR CARRYING OUT THE INVENTION

In the disinfecting method according to the present invention, the object to be disinfected, such as the medical instruments including the contact lenses and the ophthalmic contact mirrors, are immersed in the disinfecting liquid in which the hydrogen peroxide is included in an aqueous medium such as purified water, and the disinfecting liquid is irradiated with the light having a wavelength of 280-385 nm, so that the object immersed in the disinfecting liquid is disinfected. The present invention is characterized in that the disinfecting liquid including the hydrogen peroxide is accommodated in a container such that the disinfecting liquid has a depth or a thickness as small as 3-10 mm, and that the disinfecting liquid is irradiated with the light having a suitable wavelength such that the light is incident on the disinfecting liquid in a direction of the depth or the thickness of the disinfecting liquid accommodated in the container.

In the disinfecting liquid used in the present invention, the hydrogen peroxide conventionally used as the germicide is included and dissolved. With the object being immersed in the disinfecting liquid, the disinfecting liquid is irradiated with the light having the predetermined wavelength, so that the hydrogen peroxide in the disinfecting liquid is irradiated with the light. Accordingly, the hydrogen peroxide exposed to the light is effectively decomposed. Therefore, the concentration of the hydroxyradical formed upon decomposition of the hydrogen peroxide is increased within a relatively short period of time, so that the harmful microorganisms present in the disinfecting liquid can be effectively killed owing to the strong oxidative power of the hydroxyradical. Accordingly, the object immersed in the disinfecting liquid is sufficiently disinfected owing to the effective germicidal efficacy.

In irradiating the disinfecting liquid with the light as described above, the disinfecting liquid needs to be accommodated in a suitable container such that the disinfecting liquid in the container has a depth or a thickness as small as 3-10 mm, so that the hydrogen peroxide included in the disinfecting liquid can be uniformly and sufficiently irradiated with the light having the predetermined wavelength. The disinfecting liquid needs to be irradiated with the light at least in the direction of the depth or the thickness of the disinfecting liquid accommodated in the container. If the depth (thickness) of the disinfecting liquid in the container is less than 3 mm, the amount of the disinfecting liquid required for disinfecting the object is undesirably small, and the amount of the hydrogen peroxide included in the disinfecting liquid is accordingly small, resulting in an insufficient disinfecting effect. In this case, depending on the object to be disinfected, the object may not be entirely immersed in the disinfecting liquid, causing a risk that a local portion of the object which is not immersed in the disinfecting liquid is not disinfected. On the other hand, if the depth (thickness) of the disinfecting liquid in the container exceeds 10 mm, the light is not likely to be sufficiently incident on the hydrogen peroxide existing in a portion of the disinfecting liquid on the side remote from the light source, while the hydrogen peroxide existing in the other portion of the disinfecting liquid on the side near the light source is irradiated with the light. In this case, it is difficult to expect an effective disinfecting efficacy.

The container, in which the disinfecting liquid is accommodated such that the disinfecting liquid has a depth (thickness) of 3-10 mm, is not particularly limited, but may be suitably selected from among any known various containers, as long as the containers have sizes sufficient for accommodating the object to be disinfected, and permit the disinfecting liquid to have a depth or a thickness of 3-10 mm when accommodated in the containers, and as long as the containers permit the incident light to transmit therethrough in at least the direction of the depth or the thickness of the disinfected liquid accommodated therein. The container is preferably formed of a light-transmitting material, so that the light is incident on the disinfecting liquid in a plurality of directions. The disinfecting liquid accommodated in the thus formed container is irradiated with the light in the direction of its depth or thickness, and in any other directions, whereby the light irradiation efficiency is effectively increased, resulting in a further improved disinfecting effect.

The disinfecting liquid used in the present invention includes, as the germicide, the hydrogen peroxide which is an oxidizing agent. The amount of the hydrogen peroxide included in the disinfecting liquid is suitably determined depending upon the kind of the object to be disinfected and other factors, so as to obtain the desired disinfecting effect. In the present invention, the hydrogen peroxide is decomposed in a short period of time owing to the exposure to the light, for thereby increasing the concentration of the hydroxyradical in the disinfecting liquid, so that the object is disinfected owing to the strong oxidizing activity of the hydroxyradical. Accordingly, the concentration of the hydrogen peroxide included in the disinfecting liquid can be made much lower than that in the conventional arrangement wherein the hydrogen peroxide is used without being subjected to any light irradiation. For instance, the concentration of the hydrogen peroxide in the present disinfecting liquid is reduced to 1/60 or lower than that in the conventional arrangement. Accordingly, the concentration of the hydrogen peroxide in the present disinfecting liquid is held generally in a range of 1-500 ppm, preferably in a range of 3-350 ppm, and more preferably in a range of 5-50 ppm. Since the concentration of the hydrogen peroxide is made significantly low in the present invention, the hydrogen peroxide included in the disinfecting liquid is substantially entirely decomposed by exposure to the light, so that the amount of the hydrogen peroxide remaining in the disinfecting liquid without being decomposed after the exposure to the light is significantly small, eliminating the neutralizing treatment conventionally conducted after the disinfecting treatment to neutralize the residual hydrogen peroxide. Accordingly, the present arrangement enjoys the advantages of high economy and easy handling of the disinfecting liquid.

If the concentration of the hydrogen peroxide is less than 1 ppm, the disinfecting liquid may not exhibit a sufficient disinfecting effect. While the use of the hydrogen peroxide in an excessively high concentration assures a sufficient disinfecting effect, there may be a risk that the hydrogen peroxide which has not been decomposed by irradiation with the light remains in the disinfecting liquid and adheres to the disinfected object. If the disinfected object such as the medical instruments were used with the hydrogen peroxide adhering thereto, it would give an adverse influence on the living subject. In this case, the conventional neutralizing treatment needs to be conducted for neutralizing the hydrogen peroxide remaining in the disinfecting liquid.

The disinfecting liquid which includes a suitable amount of the hydrogen peroxide described above may further include a water-dispersible titanium oxide. Where the disinfecting liquid includes the water-dispersible titanium oxide in addition to the hydrogen peroxide, the disinfecting liquid exhibits a synergistically enhanced germicidal effect higher than a disinfecting liquid which contains only one of the hydrogen peroxide and the titanium oxide, owing to the germicidal effect exhibited by the titanium oxide based on its photocatalytic action and the germicidal effect exhibited by the hydrogen peroxide. Therefore, the disinfecting liquid exhibits an advantageously increased disinfecting effect with respect to the object. Like the hydrogen peroxide, the titanium oxide forms the hydroxyradical by exposure to the light, resulting in a considerably efficient reaction for decomposing organic matters such as harmful microorganisms.

The water-dispersible titanium oxide is not particularly limited as long as it can be dispersed in a suitable aqueous medium. It is preferable to employ the titanium oxide having an average particle size of generally not greater than 15 nm, preferably not greater than 10 nm. If the particle size of the titanium oxide is excessively large, the disinfecting liquid does not have a sufficiently high degree of transparency. In this case, the light is prevented from transmitting through the disinfecting liquid by the fine particles of the titanium oxide, existing in a portion of the disinfecting liquid on the side near the light source. Therefore, the fine particles of the titanium oxide existing in the other portion of the disinfecting liquid on the side remote from the light source are prevented from being irradiated with the light, causing undesirable problems such as a considerably large reduction of the disinfecting effect. The water-dispersible titanium oxide used as the component for improving the disinfecting effect is not likely to be adsorbed on the object to be disinfected. Accordingly, the addition of the titanium oxide to the disinfecting liquid does not cause problems of an undesirable change of the properties and the configuration of the object to be disinfected and an adverse influence on the living subject.

The disinfecting liquid employed in the present invention may include a metal ion, as needed. The addition of the metal ion causes a Fenton reaction between the hydrogen peroxide and the metal ion, so as to promote the decomposition of the hydrogen peroxide. Accordingly, the concentration of the hydroxyradical in the disinfecting liquid is increased, so that the disinfecting liquid exhibits a further enhanced germicidal activity. The metal ion to be added to the disinfecting liquid is not particularly limited, but may be suitably selected from among an iron ion, a nickel ion, and a copper ion, for instance. Among those metal ions, the iron ion assures a particularly high effect of promoting the decomposition of the hydrogen peroxide. Therefore, the iron ion added to the disinfecting liquid effectively promotes the decomposition of the hydrogen peroxide, and contributes to an improvement in the germicidal efficacy of the disinfecting liquid. The metal ion is added to the disinfecting liquid generally in an amount of 1-100 ppm.

The metal ion described above may be added to the disinfecting liquid as a metal-EDTA complex such as an Fe-EDTA complex, an Ni-EDTA complex, or a Cu-EDTA complex. Like the metal ion described above (especially the iron ion), the metal-EDTA complex promotes the decomposition of the hydrogen peroxide, for thereby improving the germicidal efficacy. It is preferable that the metal-EDTA complex is included in the disinfecting liquid generally in a concentration of 10-300 ppm.

The present disinfecting liquid may further contain a surfactant so that the disinfecting liquid has an activity for cleaning the object to be disinfected, in addition to the germicidal activity for killing the microorganisms. The surfactant to be added to the present disinfecting liquid may be suitably selected from among known anionic, nonionic, amphoteric, and cationic surfactants, which are generally used in the conventional contact lens liquid agents. Owing to the addition of the surfactant, the disinfecting liquid exhibits a cleaning effect such as removal of lipid stains from the object to be disinfected.

In addition to the hydrogen peroxide, titanium oxide, metal ion, metal-EDTA complex, and surfactant, the present disinfecting liquid may further include, as needed, various known additives as usually used in the conventional disinfecting liquid. For instance, the present disinfecting liquid may include any combination of a chelating agent, a buffer, a thickener, a preservative, a germicide, an oxidizing agent, and a tonicity adjusting agent. The additives to be included in the disinfecting liquid should be safe to the living subject and should not give an adverse influence on the effects provided by the hydrogen peroxide. The additives are included in the disinfecting liquid in amounts that do not inhibit the effects provided by the hydrogen peroxide.

The chelating agent to be included as the additive in the disinfecting liquid is effective to prevent metal ions such as a calcium ion from depositing on the object to be disinfected. Examples of the chelating agent include ethylenediamine tetraacetic acid, citric acid, gluconic acid, tartaric acid, phytic acid and salts thereof (e.g., sodium salt). The buffer to be included in the disinfecting liquid adjusts a pH of the disinfecting liquid to an appropriate level for the purpose of maintaining the physical properties and the configuration of the object to be disinfected. Typical examples of the buffer are citric acid, malic acid, lactic acid, ascorbic acid, maleic acid, gluconic acid, phosphoric acid, boric acid, amino acid such as glycine or glutamic acid, tris(hydroxymethyl)aminomethane, and salts of those acids (e.g., sodium salt).

As the thickener used to adjust the viscosity of the disinfecting liquid, a suitable viscous base is employed such as polyvinyl alcohol, poly-N-vinylpyrrolidone, polyethylene glycol, polyacrylamide, a hydrolyzed product of polyacrylamide, polyacrylic acid, hydroxyethyl cellulose, carboxymethyl cellulose, methylhydroxyethyl cellulose, methylhydroxypropyl cellulose, methyl cellulose, gelatin, sodium alginate, sodium chondroitin sulfate, xanthan gum, gum Arabic, or guar gum. The disinfecting liquid may include, as the preservative/germicide or the oxidizing agent, various known compounds which exhibit the preservative or germicidal efficacy, in addition to the hydrogen peroxide employed in the present invention. Examples of the oxidizing agent include peroxides such as ozone water, sodium peroxide, magnesium peroxide, and silver oxide. Owing to the addition of the oxidizing agent (peroxide), the disinfecting liquid exhibits a further improved disinfecting effect.

The present disinfecting liquid preferably has an osmotic pressure of 250-350 mOsm, for protecting the living subject from being adversely influenced when the disinfected object is used with the disinfecting liquid remaining on and adhering to its surface. To adjust the osmotic pressure of the disinfecting liquid to a desired level, a suitable amount of various known tonicity adjusting agents such as sodium chloride or potassium chloride is preferably added to the disinfecting liquid. Where the sodium chloride is used as the tonicity adjusting agent, the amount of the sodium chloride to be added is preferably in a range of 0.7-1.2 wt.%, and more preferably in a range of 0.8-1.1 wt.%, for the total weight of the disinfecting liquid. Where the contact lens is disinfected by using the present disinfecting liquid, the irritation to the eye of the user is advantageously reduced since the osmotic pressure of the disinfecting liquid is substantially equal to that of the tear fluid, even if the contact lens is worn on the eye with the disinfecting liquid adhering to its surface and the disinfecting liquid gets in the eye.

As the aqueous medium in which various components described above are included, there may be employed a solution which is principally constituted by water, in addition to tap water, purified water, and distilled water. Such a solution includes a saline solution, and a known aqueous liquid agent such as a contact lens storing liquid or a cleaning liquid.

In the present invention, the disinfecting liquid in which various components described above are included is accommodated in a suitable container such that the disinfecting liquid accommodated in the container has a depth or a thickness of 3-10 mm, and the object to be disinfected is immersed in the disinfecting liquid. In this state, the light having the predetermined wavelength is incident on the disinfecting liquid at least in the direction of the depth or the thickness of the disinfecting liquid accommodated in the container, so that the object immersed in the disinfecting liquid is disinfected.

As the light to be incident on the disinfecting liquid in which the object is immersed, there may be employed an ultraviolet (UV) radiation having a wavelength generally in a range of 250-385 nm, preferably in a range of 280-385 nm, and more preferably in a range of 320-380 nm, for thereby advantageously promoting the decomposition reaction of the hydrogen peroxide. The light is not particularly limited as long as the dominant wavelength of the light is held in the specified range described above. For instance, a natural light, a UV radiation emitted from a suitable light exposure device or a light emitting device, and a UV radiation emitted from an incandescent lamp or a fluorescent lamp can be employed. If the wavelength of the light to be incident on the disinfecting liquid is less than 250 nm, there may be a risk that the object immersed in the disinfecting liquid suffers from an adverse influence. For instance, if the object is a contact lens, the contact lens may suffer from deterioration. In view of this, it is preferable to avoid a use of the light whose wavelength is less than 250 nm. As well known, since the hydrogen peroxide has low stability with respect to the light, the hydrogen peroxide is decomposed by exposure to the light whose wavelength is larger than 385 nm. For obtaining an excellent disinfecting effect, however, the light whose dominant wavelength is held in the range of 250-385 nm is employed in the present invention.

Since the hydrogen peroxide has a low stability with respect to the light as described above, the decomposition reaction of the hydrogen peroxide is advantageously promoted by exposure to the light having a relatively low intensity. It is, however, preferable that the light to be incident on the disinfecting liquid has an intensity of 0.3-1.0 mW/cm$^2$ at its dominant wavelength. If the intensity of the light is excessively low, the hydrogen peroxide is not sufficiently decomposed, so that the intended disinfecting effect can not be attained. On the contrary, the excessively high light intensity may give an adverse influence on the object immersed in the disinfecting liquid. For instance, the contact lens may suffer from deterioration such as a change of its color to yellow, or a reduction in its UV-absorbing power.

The period of time during which the object is disinfected by the disinfecting liquid that is irradiated with the light described above is suitably determined depending upon a desired degree of disinfection of the object, by taking account of various factors such as the kind of the object to be disinfected, the wavelength and intensity of the light to be employed. For obtaining the desired disinfecting effect to a sufficient extent, the disinfecting liquid needs to be irradiated with the light generally for at least 30 minutes. For improving the efficiency of the disinfecting operation, the disinfecting liquid is irradiated with the light for up to about 12 hours, preferably up to about 6 hours.

According to the procedure described above, the object can be effectively disinfected in a simplified manner without any trouble in handling the disinfecting liquid, by simply irradiating the disinfecting liquid including the hydrogen peroxide with the light having the predetermined wavelength, for the predetermined time period.

The object which has been disinfected according to the present method is used after it is taken out of the disinfecting liquid. Where the disinfecting liquid includes the hydrogen peroxide in a concentration that is held within the above-described range (1-500 ppm), the amount of the hydrogen peroxide remaining in the disinfecting liquid without being decomposed after the exposure to the light is extremely small, e.g., almost zero. Accordingly, it is not necessary to conduct the neutralizing treatment for neutralizing the hydrogen peroxide after the disinfecting operation. Therefore, the disinfected object may be used without any subsequent treatment, or may be rinsed with a saline, for instance, before the object is used for a specific purpose.

The object to be disinfected by the disinfecting liquid according to the present invention includes the medical instruments, particularly contact lenses and contact mirrors for the ophthalmic use. In particular, the contact lenses can be considerably effectively disinfected according to the present invention. The kinds of the contact lenses to be disinfected according to the present invention are not particularly limited. For instance, the present invention can be applied to low-water-content and high-water-content soft contact lenses, and hard contact lenses, irrespective of the materials of those contact lenses.

EXAMPLES

To further clarify the concept of the present invention, some examples of the invention will be described. It is to be understood that the invention is not limited to the details of the illustrated examples and the foregoing description, but may be embodied with various changes, modifications and improvements, which may occur to those skilled in the art without departing from the scope of the invention.

-Preparation of disinfecting liquid specimens-

Initially, various disinfecting liquid specimens Nos. 1-10 were prepared in the following manner. The hydrogen peroxide ($H_2O_2$), and at least one of a titanium oxide ($TiO_2$) in the form of fine particles having an average particle size of 5 nm, an Fe ion, and an Fe-EDTA complex were mixed in respective concentrations indicated in the following TABLE 1, with a separately prepared sterile saline solution (NaCl aqueous solution) containing sodium chloride (NaCl) in a concentration of 0.9 wt.%. It was confirmed that the thus prepared disinfecting liquid specimens Nos. 1-10 had osmotic pressure values in a range of 280-290 mOsm. The Fe ion was introduced into the disinfected liquid specimen by dissolving iron (III) chloride while the Fe-EDTA complex was introduced in the disinfecting liquid specimen by dissolving EDTA Fe•Na.

## TABLE 1

| | | \multicolumn{4}{c}{Concentration [ppm]} | Depth of disinfecting liquid [mm] | Intensity of light [mW/cm²] |
|---|---|---|---|---|---|---|---|
| | | $H_2O_2$ | $TiO_2$ | Fe ion | Fe-EDTA complex ion | | |
| Specimen No. | 1 | 30 | – | – | – | 7 | 0.42 |
| | 2 | 300 | – | – | – | 7 | 0.42 |
| | 3 | 300 | 15 | – | – | 7 | 0.42 |
| | 4 | 30 | – | – | – | 7 | 0.80 |
| | 5 | 30 | 15 | – | – | 7 | 0.80 |
| | 6 | 300 | – | – | – | 7 | 0.80 |
| | 7 | 300 | 15 | – | – | 7 | 0.80 |
| | 8 | 300 | 15 | 10 | – | 7 | 0.80 |
| | 9 | 30 | – | – | 100 | 7 | 0.42 |
| | 10 | 300 | – | – | – | 15 | 1.21 |

-Test for examining the disinfecting effect-

Each of the thus prepared disinfecting liquid specimens was examined of its disinfecting (germicidal) effect in the following manner. Initially, the disinfecting liquid specimens were put into respective sterile plates each having an inside diameter of 9 cm, such that the disinfecting liquid specimens accommodated in the respective plates had respective depth values as indicated in the above TABLE 1. Then, *S.a.* (*Serratia marcescens:ATCC* 13880) as the test bacterium was inoculated into the respective disinfecting liquid specimens at about $10^6$ cfu/mL. Each of the disinfecting liquid specimens was irradiated, for 3 hours, with a UV light having a wavelength of 365 nm and emitted from a UV lamp, such that the UV light was incident on the upper side of the plate in a direction of the depth of the disinfecting liquid specimen accommodated in the plate. Further, there was measured the intensity of the light incident on the surface of each disinfecting liquid specimen, at a wavelength range around 365 nm. The results of the measurement are also indicated in the above TABLE 1.

Viable cell counts were measured for the disinfecting liquid specimens which had been irradiated with the UV light, in the following manner. A predetermined amount of the disinfecting liquid specimens was taken out of the respective plates, and diluted with a sterile saline solution, so as to provide diluted samples. The viable cell counts for 1 mL of the thus obtained diluted samples were respectively measured according to a plate method. On the basis of the measured viable cell counts, there were calculated viable cell counts per 1 mL of the disinfecting liquid specimens after irradiation with the light. Then, an amount of reduction of the bacteria was calculated in logarithm (log reduction) according to the following equation. The results of calculation are indicated in the following TABLE 2.

Bacteria reduction amount [in logarithm]=

log (viable cell count per 1 mL of each specimen

immediately after inoculation) -

log (viable cell count per 1 mL of each specimen

after irradiation with the light)

## TABLE 2

| Specimen No. | log reduction |
|---|---|
| 1 | 2.8 |
| 2 | 4.7 |
| 3 | 4.8< |
| 4 | 4.7 |
| 5 | 4.7< |
| 6 | 4.7 |
| 7 | 4.7< |
| 8 | 4.7< |
| 9 | 3.2 |
| 10 | 1.1 |

As is apparent from the results indicated in the above TABLE 2, in each of the disinfecting liquid specimens Nos. 1-9 which had been irradiated with the light according to the present invention, the log reduction value indicative of the amount of reduction of the bacteria was relatively large, so that the disinfecting liquid specimens according to the present invention exhibited a high degree of germicidal efficacy. Accordingly, it is recognized that a high degree of disinfecting effect can be obtained by irradiating, with the light, the present disinfecting liquid in which the object to be disinfected is immersed.

The disinfecting liquid specimen No. 10, on the contrary, did not exhibit a sufficient disinfecting effect even if the intensity of the light incident thereon was larger than those of the light incident on the specimens Nos. 1-9 since the depth of the disinfecting liquid specimen No. 10 accommodated in the plate was larger than 10 mm.

INDUSTRIAL APPLICABILITY

As is apparent from the foregoing description, the disinfecting method according to the present invention uses the disinfecting liquid in which the hydrogen peroxide is contained in the aqueous medium. In the present method, the disinfecting liquid is accommodated in a suitable container such that the disinfecting liquid accommodated in the container has a relatively small depth of 3-10 mm, and the light having a wavelength of 280-385 nm is incident on the disinfecting liquid in the direction of its thickness. According to the present method, the hydrogen peroxide included and dissolved in the disinfecting liquid is effectively and uniformly irradiated with the light. Therefore, the present method assures an excellent disinfecting effect by simply irradiating, with the light, the disinfecting liquid in which the object to be disinfected is immersed, resulting in effective disinfection of the object.

Further, the hydrogen peroxide can be effectively decomposed by exposure to the light, so that the concentration of the hydrogen oxide included in the disinfecting liquid can be made much lower than that in the conventional disinfecting liquid. In addition, the present method does not necessarily require the neutralizing treatment for neutralizing the residual hydrogen peroxide after the disinfecting treatment.

**Claims**

1. A method of disinfecting an object by using a disinfecting liquid in which hydrogen peroxide is included in an aqueous medium, **characterized by** comprising: accommodating said disinfecting liquid in a predetermined container such that said disinfecting liquid in said container has a depth of 3-10 mm; and irradiating said disinfecting liquid in which said object to be disinfected is immersed, with a light having a wavelength of 280-385 nm, such that said light is incident on said disinfecting liquid at least in a direction of said depth of said disinfecting liquid.

2. A method according to claim 1, wherein said light to be incident on said disinfecting liquid has an intensity of 0.3-1.0 $mW/cm^2$ at a dominant wavelength thereof.

3. A method according to claim 1, wherein said hydrogen peroxide has a concentration of 1-500 ppm.

4. A method according to claim 1, wherein said disinfecting liquid has an osmotic pressure of 250-350 mOsm.

5. A method according to claim 1, wherein said disinfecting liquid further includes a surfactant.

6. A method according to claim 1, wherein said object to be disinfected is a contact lens.

7. A method according to any one of claims 1-6, wherein said disinfecting liquid further includes a water-dispersible titanium oxide.

8. A method according to any one of claims 1-6, wherein said disinfecting liquid further includes a metal ion or a metal-EDTA complex.

9. A method according to any one of claims 1-6, wherein said disinfecting liquid further includes a water-dispersible titanium oxide, and a metal ion or a metal-EDTA complex.

## INTERNATIONAL SEARCH REPORT

| International application No. |
| --- |
| PCT/JP02/08341 |

| A. CLASSIFICATION OF SUBJECT MATTER |
| --- |
| Int.Cl⁷  A61L2/18, A61L2/10 |

According to International Patent Classification (IPC) or to both national classification and IPC

| B. FIELDS SEARCHED |
| --- |
| Minimum documentation searched (classification system followed by classification symbols)<br>    Int.Cl⁷  A61L2/10, A61L2/18 |

Documentation searched other than minimum documentation to the extent that such documents are included in the fields searched

| | | | |
| --- | --- | --- | --- |
| Jitsuyo Shinan Koho | 1940–1996 | Toroku Jitsuyo Shinan Koho | 1994–2002 |
| Kokai Jitsuyo Shinan Koho | 1971–2002 | Jitsuyo Shinan Toroku Koho | 1996–2002 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| X<br>Y | WO 80/01457 A1  (PEEL, John, Longley),<br>24 July, 1980 (24.07.80),<br>Full text<br>& JP 56-500058 A      & US 4289728 A1<br>& JP 60-172666 A | 1<br>2-9 |
| Y | JP 61-142130 A  (Toppan Printing Co., Ltd.),<br>30 June, 1986 (30.06.86),<br>Full text<br>(Family: none) | 1,2 |
| Y | JP 11-33537 A  (Fuji Photo Film Co., Ltd.),<br>09 February, 1999 (09.02.99),<br>Full text; all drawings<br>(Family: none) | 3,5,7-9 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 30 October, 2002 (30.10.02) | 12 November, 2002 (12.11.02) |

| Name and mailing address of the ISA/<br>    Japanese Patent Office | Authorized officer |
| --- | --- |
| Facsimile No. | Telephone No. |

Form PCT/ISA/210 (second sheet) (July 1998)

**INTERNATIONAL SEARCH REPORT**

International application No.

PCT/JP02/08341

| C (Continuation). DOCUMENTS CONSIDERED TO BE RELEVANT | | |
|---|---|---|
| Category* | Citation of document, with indication, where appropriate, of the relevant passages | Relevant to claim No. |
| Y | WO 91/04060 A1 (Tomei Sangyo Kabushiki Kaisha), 04 April, 1991 (04.04.91), Full text; all drawings & JP 3022989 B2 & US 5246552 A1 & EP 0445330 A1 | 4,6 |
| Y | WO 96/05869 A1 (Ciba-Geigy AG), 29 February, 1996 (29.02.96), Full text & JP 10-504482 A & US 5618492 A1 & EP 0777501 A1 | 1,6 |
| Y | JP 56-75158 A (Dainippon Printing Co., Ltd.), 22 June, 1981 (22.06.81), Full text; all drawings & US 4366125 A1 | 1-9 |
| Y | JP 56-113530 A (Dainippon Printing Co., Ltd.), 07 September, 1981 (07.09.81), Full text; all drawings (Family: none) | 1-9 |
| Y | EP 0045201 A (Novus Corp. N.V.), 03 February, 1982 (03.02.82), Full text & JP 57-110252 A | 1-9 |
| Y | WO 99/08932 A1 (Tetra Laval Holdings & Finance S.A.), 25 February, 1999 (25.02.99), Full text; all drawings & JP 11-59629 A & US 6039922 A1 | 1-9 |

Form PCT/ISA/210 (continuation of second sheet) (July 1998)